# EUROPEAN PATENT SPECIFICATION

(11) **EP 0 672 252 B1**
(45) Date of publication and mention of the grant of the patent: **03.09.1997**
(21) Application number: 94918229.9
(22) Date of filing: 03.12.1993
(51) Int. Cl.: G01N 33/53, G01N 33/537, G01N 33/58

(54) **IMMUNOASSAY**
IMMONOASSAY
DOSAGE IMMUNOLOGIQUE

(30) Priority: 04.12.1992 GB 9225354
(43) Date of publication of application: 20.09.1995
(73) Proprietor: THE VICTORIA UNIVERSITY OF MANCHESTER, Manchester M13 9PL (GB)
(72) Inventor: TRELOAR, Paul, Howard, Chester CH3 5LL (GB); KANE, John, William, Boothstown, Worsley, Manchester M28 4LX (GB); VADGAMA, Pankaj, Maganlal, Worsley, Manchester M28 4QA (GB)
(74) Representative: Atkinson, Peter Birch
(86) International application number: GB9302491
(87) International publication number: WO9414066

(56) References cited:
- EP-A- 0 097 952
- EP-A- 0 167 248
- EP-A- 0 500 305
- WO-A-90/05910
- WO-A-92/16647

## Description

The present invention relates to an immunoassay method.

Immunoassay is used to determine the amount of a given analyte species in a sample. The analyte species being determined may, for example, be a drug in a physiological fluid such as blood. The immunoassay method utilises
(i) an antibody (Ab) which will bind with the analyte (Ag) being determined, and
(ii) a labelled analyte (Ag*) which, in competition with the analyte (Ag), will also bind with the antibody (Ab).

The labelled analyte (Ag*) may for example be the same as (or an analogue) of the analyte (Ag) but provided with a radiolabel or fluorescent label.

Given that the amounts of antibody (Ab) and labelled analyte (Ag*) used in the immunoassay are known and also that the amount of the labelled analyte (Ag*) which binds to the antibody can be determined by virtue of the presence of the label, it is possible to determine the amount of the analyte (Ag) in the original sample. Such techniques are well known in the art.

Significant effort has been applied to development of electrochemical immunoassays in the last decade, owing to an active search for nonisotopic alternatives to radioimmunoassay. Electrochemical detection offers notable advantages including the ability to analyse turbid solutions (e.g. whole blood or serum) with high sensitivity. Of the electrochemical immunoassays which have been developed to date, the majority employ enzyme labels which are detected by converting an electroinactive substrate to an electroactive product. Although this approach has the benefit of enzyme amplification, the enzyme may be more labile than a direct electrochemical label. Also, an additional reagent (the enzyme substrate) is required and the approach of end-point analysis typically employed, complicates assay protocol and extends assay time.

WO-A-92/16647 discloses a sensor in which permselective membrane forms a barrier between a sample to be analysed and the detector (an electrochemical sensor). The membrane allows to pass therethrough a preselected lipophilic component which is either electrochemically active itself or which interacts with, for example, an enzyme to produce a detectable electrochemically active species. The specification further discloses a technique in which an antigen (Ag) and an antigen (Ag*) conjugated to an enzyme react competitively with an antibody. The active enzyme in the unbound antigen Ag* acts on a substrate which interacts further, either directly or with further components, to produce a lipophilic product which can pass through the permselective medium to the sensor and produce an electrochemical signal at the sensor surface.

WO-A-90/05910 discloses a biosensor which is used in an assay procedure in which one member of a specific binding pair (the other being the analyte of interest) is immobilised on to the sensor and a substrate converter (e.g. an enzyme) is conjugated to the analyte. After the competition reaction has taken place, the enzyme hydrolyses a substrate which then participates in a reaction to produce changes in the electroactive species H₂O₂ or O₂ which are electrochemically detected at the sensor.

According to the present invention there is provided an electrochemical immunoassay method for determining the amount of a first analyte in a sample comprising treating the sample with (i) a second labelled analyte which is electrochemically detectable, and (ii) an antibody which will competitively bind with the first and second analytes wherein electrochemical detection of unbound second labelled analyte is effected at an electrode separated from the sample by a membrane which permits passage of the unbound second labelled analyte therethrough but not its complex with the antibody.

The use of the labelled analyte which is capable of electrochemical determination has the advantage that it provides a direct electrochemical immunoassay without the need for an enzyme label. The use of the membrane is a particularly convenient way of effecting separation of the unbound and bound fractions of the labelled analyte.

Preferably the labelled analyte is electrochemically detectable by anodic oxidation, and the detection is effected by oxidation at an anode (separated from the sample by the membrane). The use of a label which is detected by anodic oxidation has the advantage that there is no interference by atmospheric oxygen and therefore there is no need to take special precautions to exclude the presence of oxygen or to deoxygenate the sample.

Preferably the label compound is capable of being anodically oxidised at a potential of less than +1.2 v (vs Ag/AgCl) and greater than 0 v.

The electrochemical detection method used is preferably an amperometric technique.

It is also preferred that the labelled analyte is fluorescent as well as being electrochemically detectable. This has the advantage that assay development is greatly facilitated as the immunoassay can be optimised by fluorescent measurement prior to electrochemical detection. Results from electrochemical measurements can be verified by comparison against a standard fluorometric method. Electrochemical detection of the same assay can then be performed without requiring further modification of assay conditions, and the results verified by fluorescent detection. Although direct electrochemical labels have previously been used for immunoassay, none have allowed such a simple control using an alternative detection technique. This is particularly useful given the high sensitivity of electrochemical detection required, and the artifacts of the technique which may initially be mistaken as antibody binding of the label.

Preferred label compounds for use in the invention are those which have at least one hydroxyl and/or amino group attached to an aromatic nucleus as such compounds are generally electrochemically active. The label compound should also be one which can easily be reacted to form the labelled analyte to be used in the immunoassay.

The preferred label compound is one which provides a fluorescein residue in the labelled antigenic species. Fluorescein is a compound of the formula 1 which undergoes anodic oxidation at >+0.65V (vs Ag/AgCl) in accordance with the equation shown below.

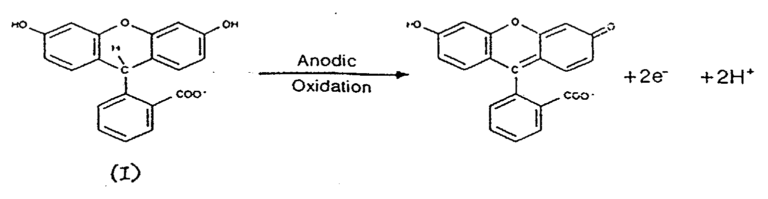

A labelled antigenic species incorporating a fluorescein residue may conveniently be prepared by reaction of fluorescein isothiocyanate (FITC) with an analyte component which may for example be the same as (or an analogue of) the analyte to be determined.

Alternatively, the fluorescent residue may be provided by rhodamine.

In accordance with the invention, a membrane is used to separate the free and bound fractions of the labelled antigenic species so that electrochemical detection (preferably by anodic oxidation) of the unbound labelled analyte may be effected. The membrane will be such as to allow the unbound labelled analyte to pass therethrough (but not its higher molecular weight complex with the antibody) so that the unbound fraction passes through to the detecting electrode thereby providing a current which is representative of the amount of the unbound labelled species. From this information, the amount of the analyte being determined may be calculated by methods well-known to those skilled in the art. Typically the membrane will only allow the passage of molecules having a molecular weight of less then 100,000.

It is also within the scope of the invention for the antibody to be bound to a particulate support material (having a size of up to, say, 10 µm (microns) and for the labelled analyte to have a molecular weight higher than 100,000. Thus the labelled analyte may have a molecular weight of say up to 200,000 in which case the membrane will be such as to allow passage of molecules having a molecular weight of less than 200,000. Such a membrane will preclude passage of antibody bound to the particulate support.

Generally the membrane will also permit passage therethrough of the unbound analyte being determined (but not its complex with the antibody) and therefore the analyte to be determined should not be electrochemically active, or at least not electrochemically active at the same potential, as the labelled analyte.

The analyte being determined and the labelled analyte may be lipophilic. The membrane may be a permselective membrane which is substantially impermeable to charged substantially non-lipophilic species and which allows separation of the free labelled lipophilic analyte from the antibody bound fraction. With such a membrane, the method of the invention may be used for determining the amounts of an analyte in a fluid sample which contains substantial amounts of hydrophilic components and ionised species which would oxidise at the anode and foul the electrode. In other words, the hydrophilic components and ionised species would otherwise be interferents in the immunoassay. For example, in the case of physiological fluids, the analyte to be determined may be a drug and interferents commonly encountered using amperometric sensors are charged ascorbate, urate, glutathione and certain amino acids. The use of the membrane permits the immunoassay of the invention to be effected on physiological fluids without interference from the aforementioned components. Furthermore the immunoassay may be performed on a turbid liquid (e.g. blood) without clarification thereof thereby avoiding the need for sample preparation.

The permselective membrane may for example be of the type described in PCT Application No. ACT/GB 92/00443. Such membranes comprise a permselective medium and means of immobilising this medium as a continuous liquid or gel phase to form a substantially electrically insulating barrier between the electrode and the sample. The membrane may comprise a polymeric material incorporating a plasticiser which is substantially miscible with the polymeric material to form a substantially homogeneous phase in which the plasticiser is immobilised by the polymeric compounds. A suitable polymeric material is polyvinyl chloride and examples of plasticisers are di-octyl phthalate (DOP) and iso-propyl myristate (IPM). Other plasticisers such as diphenyl ether and dioctyl adipate may also be suitable. Such membranes are particularly suitable because they are biocompatible and are not fouled by blood or other physiological fluids.

Examples of other membranes which may be used include polyurethane, cellulose acetate, and polyether sulphone membranes as well as Diamond Like Carbon (DLC) coated membranes.

If desired, the membrane may be supported on a microporous support, e.g. of Cuprophan.

It will be appreciated from the foregoing description that the inventions allow rapid immunoassay on, for example, whole blood samples with no sample preparation and involving addition of only a small volume sample to an immunosensor. Therefore it is possible for homogeneous assays using simple, cheap, robust and portable instrumentation to be carried out by unskilled operators enabling decentralised testing.

The invention will be further described by way of example only with reference to the accompanying drawings, in which:
Fig. 1 illustrates an electrochemical immunoassay embodying membrane separation;
Fig. 2 is a graph comparing immunoassay results for phenytoin obtained using electrochemical and fluorescent detection; and
Fig. 3 is a comparison of results obtained in immunoassay procedures using two different methods of detection.

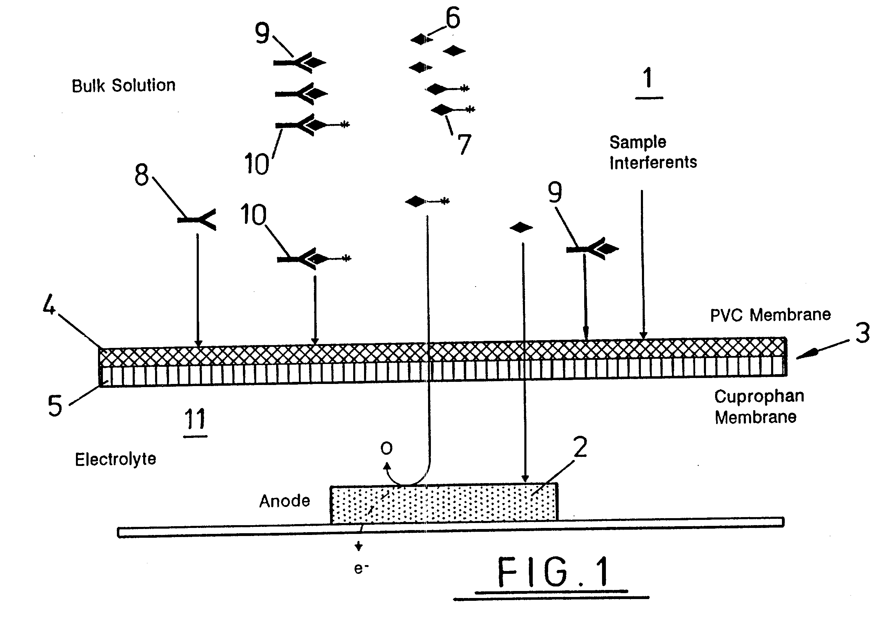

As shown in Fig. 1, a fluid sample 1 is shown as being separated from an anode 2 by a membrane assembly 3 which comprises a permselective PVC membrane 4 associated with a microporous support in the form of a Cuprophan membrane 5. The PVC membrane is of the type discussed above and is substantially impermeable to charged substantially non-lipophilic species. Furthermore, as described below, the PVC membrane 4 allows analyte species (both labelled and unlabelled) and labelled antigen to pass across the membrane but not analytelantibody complexes.

The sample 1 (which may for example be a physiological fluid such as blood) is shown as containing an analyte 6 whereof the amount in the sample is to be determined by the immunoassay procedure. The analyte 6 may for example be an anti-epileptic drug. Also shown in the sample 1 are a labelled analyte species 7 and an antibody 8 which is capable of competitively binding with the analyte 6 and labelled analyte 7 to form conjugates 9 and 10 respectively.

The label is one which is capable of electrochemical detection and may, for example, be provided by a fluorescein residue.

An electrolyte 11 is provided between the membrane 2 and anode 3.

The only species which are able to pass across the membrane are the analyte 6 to be determined and the labelled analyte 7. Sample interferents as well as the antibody, and the antibody conjugates are not able to pass across the membrane.

The analyte 6 is electrochemically inactive while the labelled analyte 7 is electrochemically active and is oxidised at the anode resulting in an electric current which is measured to determine the amount of the unbound analyte 7. It is thus possible to determine the amount of the analyte 6. It will be appreciated that analysis of turbid solution is possible and there is no need to exclude oxygen.

To illustrate the effectiveness of a fluorescein residue as an electrochemical label, fluorescent detection of the label was used to develop and optimise an immunoassay for phenytoin prior to electrochemical detection. Drug antibody was precipitated with a second antisera and the solid phase antibody washed, and incubated with a FITC labelled drug analogue and drug standard. The antibody bound labelled fraction was separated by centrifugation, and unbound label activity in the assay supernatant was determined by High Performance Liquid Chromatography with electrochemical detection (HPLC-ECD).

Electrochemical detection was performed using a coulometric cell comprised of two electrodes in series. Optimum detection of the FITC label was achieved by polarising the upstream (screening electrode) at +0.75V, and the downstream (quantitating) electrode at +1.0V (vs Ag/AgCl). With this electrode configuration, a degree of electrochemical selectivity was afforded which complemented chromatographic isolation of the product.

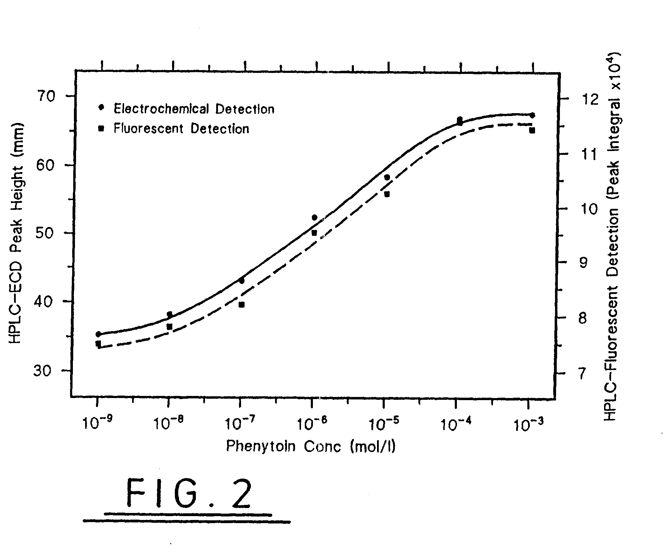

Dose response curves using this method of electrochemical detection are shown in Fig. 2 which also includes similar curves obtained by fluorescent detection. It will be seen that the curves are superimposable thus indicating the effectiveness of the electrochemical label.

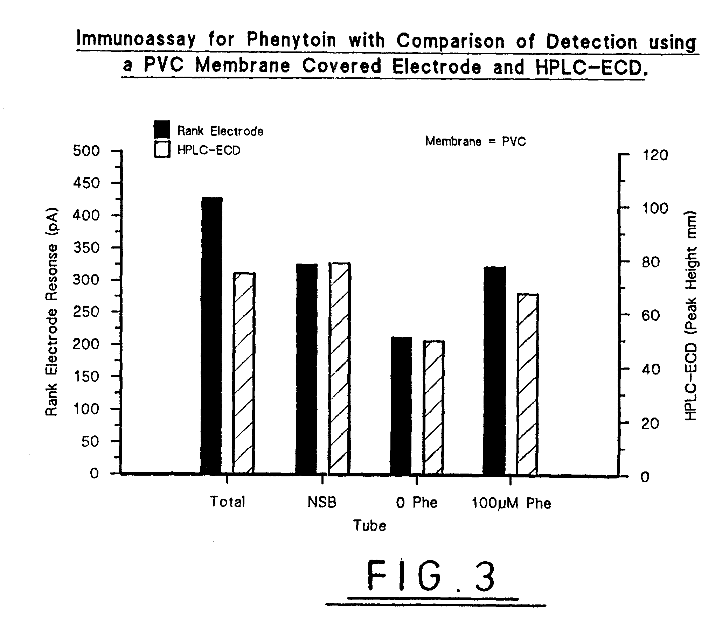

Figure 3 is a comparison of results obtained in an immunoassay for phenytoin using two different detection methods, namely (i) an electrode covered with a PVC membrane (of the type described above), and (ii) HPLC-ECD.

Four different experimental procedures were effected using each of the two detection methods. All procedures used labelled drug analogue but the procedures differed in the other components present, as set out below.

| Procedure | Component Present |
|---|---|
| "Total" | - |
| "NSB" | non-specific antibody |
| "O Phe" | specific antibody |
| "100 µm Phe" | specific antibody + phenytoin |

All experimental procedures involved centrifugation to separate analyte-antibody complexes, the detection then being effected on the supernatant. Thus, as far as the electrode covered with the PVC membrane is concerned, the procedures demonstrate the effectiveness of the membrane in allowing the unbound labelled analyte to pass through the membrane.

The first point to note is that, in any one experimental procedure, the results obtained using the two detection methods were generally similar (although in the "Total" procedure the membrane covered electrode gave a higher response than the HPLC-ECD detection - this could be an artifact).

The results obtained using the "Total" and "NSB" procedures are substantially the same thus demonstrating that there are no problems with non-specific binding and that labelled analyte can be determined with the membrane covered electrode.

The "O Phe" shows a lower amount of unbound drug analogue than either the "Total" or "NSB" procedures due to the binding of a portion of the drug analogue with the specific antibody. The fact that the results obtained in the "O Phe" procedure are identical using the two detection methods shows that the membrane is effective in allowing the passage of the labelled drug analogue.

The "100 µm Phe" procedure demonstrates a higher amount of detected labelled drug analogue than the "O Phe" procedure. This is due, of course, to the fact that in the former procedure the labelled drug analogue is in competition with the phenytoin for binding to the antibody.

## Claims

1. An electrochemical immunoassay method for determining the amount of a first analyte in a sample comprising treating the sample with (i) a second labelled analyte which is electrochemically detectable, and (ii) an antibody which will competitively bind with the first and second analytes wherein electrochemical detection of unbound second labelled analyte is effected at an electrode separated from the sample by a membrane which permits passage of the unbound second labelled analyte therethrough but not its complex with the antibody.

2. A method as claimed in claim 1 wherein the labelled analyte is electrochemically detectable by anodic oxidation, and said detection is effected by oxidation at an anode.

3. A method as claimed in claim 2, wherein the labelled analyte is capable of being anodically oxidised at a potential of less than +1.2v (vs Ag/AgCl) and greater than Ov.

4. A method as claimed in claim 2 or 3 wherein the label of the second analyte contains at least one hydroxyl and/or amino group attached to an aromatic nucleus.

5. A method as claimed in any one of claims 1 to 4 wherein the label of the second analyte is fluorescent.

6. A method as claimed in claim 5 wherein the label of the second analyte includes a fluorescein or rhodamine residue.

7. A method as claimed in any one of claims 1 to 6 wherein the membrane prevents the passage to the electrode of interferent species.

8. A method as claimed in claim 7 wherein the membrane is substantially impermeable to charged substantially non-lipophilic species.

9. A method as claimed in any one of claims 1 to 8 wherein the membrane is a permselective membrane.

10. A method as claimed in claim 9 wherein the permselective membrane is immobilised as a continuous liquid or gel phase.

11. A method as claimed in claim 10 wherein the permselective membrane comprises polyvinyl chloride incorporating a plasticiser.

12. A method as claimed in claim 11 wherein the plasticiser is dioctyl phthalate and/or iso-propyl myristate.

## Patentansprüche

1. Elektrochemische Immunoassay-Verfahren zur Bestimmung der Menge eines ersten Analyten in einer Probe, das folgendes umfaßt: Behandeln der Probe mit (i) einem zweiten markierten Analyten, der elektrochemisch nachweisbar ist, und (ii) einem Antikörper, der sich mit dem ersten und zweiten Analyten kompetitiv verbindet, wobei der elektrochemische Nachweis von ungebundenem zweitem markiertem Analyten an einer Elektrode durchgeführt wird, die von der Probe durch eine Membran getrennt ist, die den Durchgang des ungebundenen zweiten markierten Analyten, jedoch nicht dessen Komplexverbindung mit dem Antikörper, erlaubt.

2. Verfahren nach Anspruch 1, bei dem der markierte Analyt durch anodische Oxidation elektrochemisch nachweisbar ist und der Nachweis durch Oxidation an einer Anode ausgeführt wird.

3. Verfahren nach Anspruch 2, bei dem der markierte Analyt dazu fähig ist, bei einem Potential von weniger als +1,2 V (mit Bezug auf Ag/AgCl) und mehr als 0 V anodisch oxidiert zu werden.

4. Verfahren nach Anspruch 2 oder 3, bei dem die Markierung des zweiten Analyten mindestens eine an einen aromatischen Kern angelagerte Hydroxyl- und/oder Aminogruppe enthält.

5. Verfahren nach einem der Ansprüche 1 bis 4, bei dem die Markierung des zweiten Analyten fluoreszierend ist.

6. Verfahren nach Anspruch 5, bei dem die Markierung des zweiten Analyten einen Fluorescein- oder Rhodaminrest enthält.

7. Verfahren nach einem der Ansprüche 1 bis 6, bei dem die Membran den Durchgang störender Spezies zur Elektrode verhindert.

8. Verfahren nach Anspruch 7, bei dem die Membran im wesentlichen für geladene im wesentlichen nichtlipophile Spezies undurchlässig ist.

9. Verfahren nach einem der Ansprüche 1 bis 8, bei dem die Membran eine permselektive Membran darstellt.

10. Verfahren nach Anspruch 9, bei dem die permselektive Membran als geschlossene Flüssig- oder Gelphase immobilisiert ist.

11. Verfahren nach Anspruch 10, bei dem die permselektive Membran einen Weichmacher enthaltenden Polyvinylchlorid darstellt.

12. Verfahren nach Anspruch 11, bei dem der Weichmacher Dioctylphthalat und/oder Isopropylmyristat ist.

## Revendications

1. Procédé d'immunodosage électrochimique pour déterminer la quantité d'un premier analyte dans un échantillon, comprenant le fait de traiter l'échantillon avec (i) un second analyte marqué détectable par voie électrochimique et (ii) un anticorps qui va se lier de manière compétitive avec les premier et second analytes, la détection électrochimique du second analyte marqué non lié étant réalisée à une électrode séparée de l'échantillon par une membrane qui permet le passage du second analyte marqué non lié, mais non de son complexe avec l'anticorps.

2. Procédé selon la revendication 1, dans lequel l'analyte marqué est détectable par voie électrochimique à l'aide d'une oxydation anodique, ladite détection étant réalisée par oxydation à l'anode.

3. Procédé selon la revendication 2, dans lequel l'analyte marqué peut être soumis à une oxydation anodique à un potentiel inférieur à +1,2V (par rapport à Ag/AgCl) et supérieur à 0V.

4. Procédé selon la revendication 2 ou 3, dans lequel le marqueur du second analyte contient au moins un groupe hydroxyle et/ou un groupe amino fixé à un noyau aromatique.

5. Procédé selon une quelconque des revendications 1 à 4, dans lequel le marqueur du second analyte est de type fluorescent.

6. Procédé selon la revendication 5, dans lequel le marqueur du second analyte englobe un résidu de fluorescéine ou de rhodamine.

7. Procédé selon une quelconque des revendications 1 à 6, dans lequel la membrane empêche le passage en direction de l'électrode d'espèces interférentes.

8. Procédé selon la revendication 7, dans lequel la membrane est essentiellement imperméable à des espèces non lipophiles essentiellement chargées.

9. Procédé selon une quelconque des revendications 1 à 8, dans lequel la membrane est une membrane sélective par perméation.

10. Procédé selon la revendication 9, dans lequel la membrane sélective par perméation est immobilisée sous forme d'une phase liquide ou de gel en continu.

11. Procédé selon la revendication 10, dans laquelle la membrane sélective par perméation comprend du chlorure de polyvinyle englobant un plastifiant.

12. procédé selon la revendication 11, dans lequel le plastifiant est le phtalate de dioctyle et/ou le myristate d'isopropyle.
